# EUROPÄISCHE PATENTSCHRIFT

(11) **EP 2 976 373 B1**
(45) Veröffentlichungstag und Bekanntmachung des Hinweises auf die Patenterteilung: **14.10.2020**
(21) Anmeldenummer: 14711525.7
(22) Anmeldetag: 21.03.2014
(51) Int. Cl.: C08G 18/78, C07D 273/04, C08G 18/02, C08G 18/08, C08G 18/16

(54) **VERFAHREN ZUR HERSTELLUNG VON POLYISOCYANATEN UND KATALYSATOR-KIT HIERFÜR**
PROCESS FOR THE PREPARATION OF POLYISOCYANATES AND CATALYST KIT FOR SAME
PROCÉDÉ DE FABRICATION DE POLYISOCYANATES ET KIT DE CATALYSEUR CORRESPONDANT

(30) Priorität: 22.03.2013 EP 13160534
(43) Veröffentlichungstag der Anmeldung: 27.01.2016
(73) Patentinhaber: Covestro Intellectual Property GmbH & Co. KG, 51373 Leverkusen (DE)
(72) Erfinder: RICHTER, Frank, 51373 Leverkusen (DE); BRAHM, Martin, 51519 Odenthal (DE)
(74) Vertreter: Levpat
(86) Internationale Anmeldenummer: PCT/EP2014/055724
(87) Internationale Veröffentlichungsnummer: WO 2014/147231

(56) Entgegenhaltungen:
- EP-A1- 0 896 009
- EP-A1- 0 962 454
- WO-A1-2005/087828
- DE-A1- 19 824 490

## Beschreibung

Die vorliegende Erfindung betrifft ein Katalysator-Kit, umfassend einen Trimerisierungskatalysator für die asymmetrische Trimerisierung von Polyisocyanaten und ein Katalysatorgift des Trimerisierungskatalysators. Die Erfindung betrifft ferner die Verwendung eines solchen Katalysator-Kits zur Herstellung von Iminooxadiazindiongruppen enthaltenden Polyisocyanaten durch Oligomerisierung monomerer Di- und/oder Triisocyanate sowie ein Verfahren zur Herstellung von Iminooxadiazindiongruppen enthaltenden Polyisocyanaten

Die Oligo- bzw. Polymerisierung von Isocyanaten, hier zusammenfassend Modifizierung genannt, ist seit langem bekannt. Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Zur Deaktivierung der Katalystoren hat sich neben ihrer thermischen Zersetzung insbesondere die chemische "Neutralisation" (Vergiftung) zum "Abstoppen" der katalysierten Reaktion bewährt.

Eine Zusammenstellung dieser Verfahren des Standes der Technik findet sich in H. J. Laas et al., J. Prakt. Chem. 1994, 336, 185 ff.

Eine spezielle Form der Isocyanatmodifizierung die zu Produkten mit einem hohen Anteil an Iminooxadiazindiongruppen (asymmetrischen Isocyanat-Trimeren), neben den lange bekannten Isocyanuratstrukturen (bisher vereinfachend häufig nur als "Trimere" bezeichnet) in den Verfahrensprodukten führt, wird u.a. in EP-A 962455, 962454, 896009, 798299, 447074, 379914, 339396, 315692, 295926, DE 198 24 490 A1 sowie 235388 beschrieben. Als Katalysatoren hierfür haben sich (Hydrogenpoly)fluoride bewährt, bevorzugt mit quaternären Phosphoniumkationen als Gegenion. Ein Nachteil dieser Verfahren des Standes der Technik ist, dass sich die als Katalysator verwendeten Spezies teilweise unter Bildung störender Nebenprodukte zersetzen, was sich u.a. durch einen sukzessiv ansteigenden Phosphorgehalt des - in der Regel destillativ - wiedergewonnenen Monomers (Recyclat) äußert.

Zwar lassen sich derartig verunreinigte Recyclate aufreinigen, vgl. EP-A 1939171, jedoch ist eine derartige Vorgehensweise mit zusätzlichem Aufwand verbunden, den es zu vermeiden gilt.

Der Erfindung lag daher die Aufgabe zugrunde, ein Verfahren zur Herstellung hoch Iminooxadiazindiongruppen enthaltender Polyisocyanate zur Verfügung zu stellen, das nicht mit den vorgenannten Nachteilen behaftet ist. Die Katalysatoren sollen insbesondere eine bessere Stabilität im Isocyanatmilieu aufweisen und nicht bzw. im Vergleich mit Systemen des Standes der Technik weniger zur Zersetzung unter Bildung störender Nebenkomponenten neigen, die sich in den Verfahrensprodukten, insbesondere dem Recyclat, anreichern können.

Diese Aufgabe wird gelöst durch ein Katalysator-Kit, umfassend einen Trimerisierungskatalysator für die asymmetrische Trimerisierung von monomeren Di- und/oder Triisocyanaten und ein Katalysatorgift des Trimerisierungskatalysators, wobei das Katalysator-Kit dadurch gekennzeichnet ist, dass der Trimerisierungskatalysator und das Katalysatorgift jeweils einen Wassergehalt von höchstens 0,5 Gew.-% aufweisen, wobei der Trimerisierungskatalysator wenigstens ein quarternäres Phosphoniumsalz umfasst oder daraus besteht.

Ein weiterer Gegenstand der vorliegenden Erfindung betrifft die Verwendung des erfindungsgemäßen Katalysator-Kits zur Herstellung von Iminooxadiazindiongruppen enthaltenden Polyisocyanaten durch Oligomerisierung monomerer Di- und/oder Triisocyanate.

Weiterhin betrifft die vorliegende Erfindung ein Verfahren zur Herstellung von Iminooxadiazindiongruppen enthaltenden Polyisocyanaten, bei dem mindestens ein organisches Di-und/oder Triisocyanat unter Anwesenheit wenigstens eines Trimerisierungskatalysators für die asymmetrische Trimerisierung von Polyisocyanaten zur Reaktion gebracht und die Reaktion bei Erreichen eines vorgebbaren Umsetzungsgrades bezogen auf das organische Di- und/oder Triisocyanat durch Zugabe wenigstens eines Katalysatorgifts für den Trimerisierungskatalysator gestoppt wird, wobei das Verfahren dadurch gekennzeichnet ist, dass der Trimerisierungskatalysator und das Katalysatorgift jeweils einen Wassergehalt von höchstens 0,5 Gew.-% aufweisen und der Gesamtgehalt an Wasser aller Einsatzstoffe insgesamt höchstens 1 Gew.-% beträgt, wobei der Trimerisierungskatalysator wenigstens ein quarternäres Phosphoniumsalz umfasst oder daraus besteht.

Der Erfindung liegt die Erkenntnis zugrunde, dass bei der Verwendung von Katalysatoren und entsprechenden Katalysatorgiften, die auch als "Abstopper" bezeichnet werden, die vorgenannten Anforderungen erfüllt werden können, wenn die angegebenen Höchstwerte für den Wassergehalt nicht überschritten werden.

Verglichen mit der Katalyse z.B. durch quaternäre Phosphoniumsalze ohne Verwendung von Additiven, die dem Katalysator das Wasser entziehen und handelsüblicher Toluolsulfonsäure (Hydrat) als Abstopper (Bulk-Modifizierung), beobachtet man im erfindungsgemäßen Verfahren bei sonst gleichen Reaktionsbedingungen eine deutlich verbesserte Katalysatorstabilität, was sich in deutlich niedrigeren Phosphorgehalten des Recyclates äußert.

Die im erfindungsgemäßen Verfahren resultierenden monomerenarmen Iminooxadiazindiongruppen aufweisenden Polyisocyanate weisen das gleiche hohe Qualitätsniveau auf, wie die Produkte, die nach vorbeschriebenen Verfahren des Standes der Technik erhalten werden und sind von diesen analytisch in der Regel nicht unterscheidbar.

Bei Einsatz des Katalysator-Kits sollte zudem der Gesamtwassergehalt aller Einsatzstoffe, das heißt beispielsweise einschließlich der Reaktanden, 1 Gew.-% nicht überschreiten.

Keiner der eingangs genannten Schriften des Standes der Technik ist zu entnehmen, dass eine Absenkung des Wassergehaltes in den zur Iminooxadiazindionbildung bevorzugten Katalysatoren und Katalysatorgiften des Standes der Technik zu einer signifikanten Stabilisierung dieser Spezies im Isocyanatmilieu führt. In EP 962 454 wird Wasser sogar explizit als mögliches Additiv zur Herstellung von Fluorid-Ionen enthaltenden Katalysatoren aufgeführt, die für die Herstellung von Iminooxadiazindiongruppen enthaltenden Polyisocyanaten eingesetzt werden können. Da darüber hinaus Diisocyanate selbst gegenüber Wasser äußerst reaktiv sind, war vielmehr zu erwarten, dass die 'Entwässerung' des Katalysators bzw. des Katalysatorgiftes nach Kontakt mit dem zu modifizierenden Isocyanat rasch einsetzen würde und daher eine vorgelagerte Entwässerung des Katalysators bzw. Katalysatorgiftes keinen Einfluß auf die Stabilität der P-haltigen Spezies haben sollte.

Die Art und Weise, wie dem Katalysator bzw. dem Katalysatorgift das herstellungsbedingt enthaltene oder - z.B. aufgrund dessen Hygroskopie - später wieder aufgenommene, Restwasser entzogen wird (destillativ, extraktiv, durch chemische Reaktion mit einem im Verfahren unschädlichen Additiv, Adsorption etc.), ist im erfindungsgemäßen Verfahren belanglos.

Mit dem erfindungsgemäßen Modifizierverfahren ist daher eine verbesserte Methode zur Herstellung Iminooxadiazindiongruppen enthaltender Polyisocyanate in einfacher Weise zugänglich geworden.

Im Rahmen der vorliegenden Erfindung können neben dem Trimerisierungskatalysator auch weitere Katalysatoren eingesetzt werden. Zudem können auch Mischungen verschiedener Trimerisierungskatalysatoren eingesetzt werden, gewünschtenfalls mit weiteren Katalysatoren gemeinsam. Diese Konstellationen sind beispielsweise als Ausgestaltungen des Katalysator-Kits, dessen Verwendung oder des erfindungsgemäßen Verfahrens zu verstehen.

In Ausgestaltung des erfindungsgemäßen Katalysator-Kits weisen der Trimerisierungskatalysator und das Katalysatorgift jeweils unabhängig voneinander einen Wassergehalt von höchstens 0,4 Gew.-% auf, bevorzugt von höchstens 0,3 Gew.-% und besonders bevorzugt von höchstens 0,2 Gew.-%. Hierdurch kann die Bildungsrate unerwünschter Nebenprodukte weiter reduziert werden.

Der Trimerisierungskatalysator umfasst wenigstens ein quarternäres Phosphoniumsalz oder besteht daraus. Bevorzugte Trimerisierungskatalysatoren sind solche auf Basis von quarternären Phophoniumsalzen, deren Kationen der allgemeinen Formel R₄P⁺ entsprechen, wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können. Es können einzelne Phosphoniumsalze, ebenso wie Mischungen verschiedener Phosphoniumsalze oder Mischungen von Phosphoniumsalzen mit anderen, die Iminooxadiazindionbildung beschleunigenden Katalysatoren eingesetzt werden.

Besonders bevorzugte Trimerisierungskatalysatoren sind quaternäre Phosphoniumpolyfluoride der Formel R₄P⁺ F⁻·n(HF), wobei R für gleiche oder verschiedene, gegebenenfalls verzweigte, aliphatische, aromatische und/oder araliphatische C₁-C₂₀ Reste steht und gegebenenfalls zwei oder mehrere Substituenten R untereinander und mit dem Phosphoratom auch gesättigte oder ungesättigte Cyclen bilden können und n beliebige Werte zwischen 0,1 und 20 annehmen kann.

Es können einzelne Phosphoniumpolyfluoride der Formel R₄P⁺ F⁻·n(HF), ebenso wie Gemische dieser Salze oder Mischungen von Phosphoniumpolyfluoriden der Formel R₄P⁺ F⁻·n(HF) mit anderen, die Iminooxadiazindionbildung beschleunigenden Katalysatoren eingesetzt werden.

Als Katalysatorgifte, d.h. Abstopper, kommen ganz allgemein wasserfreie Säuren mit einem pKs-Wert unterhalb 3,2 (pKs-Wert der HF), bevorzugt unterhalb 1 in Frage. Das Katalysatorgift kann als einzelne Verbindung eingesetzt werden, ebenso wie eine Mischung verschiedener Katalysatorgifte. Bevorzugte Abstopper sind anorganische oder organische Säuren mit einer guten Löslichkeit in organischen Medien, die zu keinen unerwünschten Reaktionen mit den umzusetzenden Isocyanaten und/oder den Verfahrensprodukten führen. Beispielsweise geeignet sind wasserfreie HCl, bevorzugt als Lösung des Adduktes an das umzusetzende Isocyanat (Carbaminsäurechlorid) im überschüssigen Isocyanat, oder in anderer, wasserfreier Lösung, z.B. in polaren organischen Lösemitteln. Weiterhin geeignet sind saure Ester P- und S-haltiger Säuren sowie diese Säuren selbst. Als Beispiele seien genannt: Phosphorsäure, Phosphorsäuremono- sowie diester mit gleichen oder verschiedenen, gegebenenfalls verzweigten, aliphatischen, aromatischen und/oder araliphatischen C₁-C₂₀ Resten in der Esterfunktion, wie z.B.: Mono- und Dialkylphosphaten ggf. auch als Gemische der 3 vorstehend genannten Verbindungsklassen, die ggf. auch kleine Mengen der jeweiligen Triester enthalten können, wie sie z.B. bei der Umsetzung von H₃PO₄ bzw. POCl₃ mit Alkoholen oder Phenolen resultieren und z.B. unter der Handelsbezeichnung Hordaphos vertrieben werden, Schwefelsäure, Sulfon- und Sulfinsäuren und die von ihnen abgeleiteten, mindestens einbasigen Ester mit gleichen oder verschiedenen, gegebenenfalls verzweigten, aliphatischen, aromatischen und/oder araliphatischen C₁-C₂₀ Substituenten am Schwefelatom bzw. im Esterrest, wie z.B. Methansulfonsäure, Toluolsulfonsäure, Alkylbenzol- bzw. Alkylnaphthalinsulfonsäuren mit einem oder mehreren, gleichen oder verschiedenen, gegebenenfalls verzweigten, C₁-C₂₀ Substituenten am Benzol- bzw. Naphthalinring sowie mehr als eine Säurefunktion je Molekül enthaltende Derivate der vorgenannten Verbindungen, wie sie z.B. unter der Handelsbezeichnung Nacure und K-Cure vertrieben werden.

Das Katalysator-Kit kann Additive und/ oder Lösungsmittel beinhalten. Diese können auch unabhängig von dem Katalysator-Kit im Rahmen des erfindungsgemäßen Verfahrens eingesetzt werden. Dies sind beispielsweise den Wassergehalt des Katalysators nicht beeinflussende Stoffe wie Alkohole, Stabilisatoren (z.B. sterisch gehinderte Phenole oder Amine), Antioxidationsmittel etc. zu verstehen, die in der Polyurethanchemie üblicherweise Verwendung finden. Deren Wassergehalt liegt erfindungsgemäß insbesondere ebenfalls unter 0,5 Gew.-% bezogen auf diese Komponente.

Hinsichtlich des erfindungsgemäßen Verfahrens ist es vorgesehen, dass die Reaktion bei Erreichen eines vorgebbaren Umsetzungsgrades bezogen auf das organische Di- und/oder Triisocyanat durch Zugabe wenigstens eines Katalysatorgifts für den Trimerisierungskatalysator zu stoppen. Der Umsetzungsgrad kann beispielsweise 5 bis 80 Gew.-% des organischen Di- und/oder Triisocyanates betragen, insbesondere 10 bis 60 Gew.-% des organischen Di- und/oder Triisocyanates.

Das erfindungsgemäße Verfahren kann im Temperaturbereich von 0 °C bis + 250 °C, bevorzugt bei 20 bis 180 °C, besonders bevorzugt bei 40 bis 150 °C durchgeführt werden und bei beliebigen Umsetzungsgraden unterbrochen werden, bevorzugt nach den vorstehend genannten.

Der Bedarf an Trimerisierungskatalysator im erfindungsgemäßen Verfahren unterscheidet sich in der Regel nicht von dem in der Bulk-Modifizierung des Standes der Technik beobachteten. Der Trimerisierungskatalysator kann beispielweise in einem Anteil von 1 mol-ppm bis 1 mol-%, bevorzugt von 5 mol-ppm bis 0,1 mol-%, bezogen auf das organische Di- und/oder Triisocyanat, eingesetzt werden.

Der Katalysator kann im erfindungsgemäßen Verfahren unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. aliphatische oder aromatische Kohlenwasserstoffe, Alkohole, Ketone, Ester sowie Ether. Bevorzugt werden Alkohole verwendet.

Im Anschluß an die katalysierte Reaktion erfolgt bei dem erfindungsgemäßen Verfahren nach Erreichen des gewünschten Umsetzungsgrades die Desaktivierung des Trimerisierungskatalysators. Sie wird erfindungsgemäß durch Zugabe eines Katalysatorgifts, beispielsweise wie oben beschrieben, herbeigeführt.

Anschließend kann das nicht umgesetzte Monomer sowie gegebenenfalls mitverwendetes Lösemittel mit Hilfe aller bekannten Separationstechniken wie z.B. Destillation, gegebenenfalls in der speziellen Ausführungsform der Dünnschichtdestillation, Extraktion oder Kristallisation/Filtration abgetrennt werden. Selbstverständlich können auch Kombinationen zweier oder mehrerer dieser Techniken angewendet werden. Bevorzugt wird das nicht umgesetzte Monomer destillativ abgetrennt.

Sofern eine Abtrennung durchgeführt wird, weisen die erfindungsgemäßen Produkte nach der Abtrennung einen Restmonomergehalt < 0,5 %, bevorzugt < 0,3 Gew.- %, besonders bevorzugt <0,25 % auf.

Vorzugsweise wird das nicht umgesetzte Monomer jedoch abgetrennt. Soll beispielsweise das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordesaktivierung insbesondere in solchen Fällen auf die Monomerenabtrennung verzichtet werden.

Nach einer besonderen Ausführungsform des erfindungsgemäßen Verfahrens kann die Oligomerisierung in einem Rohrreaktor vorgenommen werden. Hierbei profitiert man ebenfalls von der geringeren Zersetzungstendenz der erfindungsgemäßen Katalysatoren. Zudem ist diese Verfahrensvariante vorteilhaft, weil diese ein kontinuierliches Arbeiten ermöglicht.

Zur Durchführung des erfindungsgemäßen Verfahrens können prinzipiell alle bekannten (Di)isocyanate des Standes der Technik einzeln oder in beliebigen Abmischungen untereinander eingesetzt werden.

Insbesondere seien genannt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

Bevorzugt werden eingesetzt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI) sowiel,3- sowie 1,4-Bis(isocyanatomethyl)cyclohexan (H6XDI).

Hierbei ist es belanglos, nach welchen Verfahren die vorstehend genannten (Poly)isocyanate generiert werden, d.h. mit oder ohne Verwendung von Phosgen.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte auf Isocyanuratbasis.

Sie können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Die nachfolgenden Vergleichsbeispiele und Beispiele sollen die Erfindung näher erläutern, ohne sie jedoch einzuschränken.

### Beispiele

Alle Mengenangaben beziehen sich, soweit nicht anders vermerkt, auf die Masse.

Die Ermittlung des NCO-Gehaltes der in den Beispielen und Vergleichsbeispielen beschriebenen Harze erfolgte durch Titration gemäß DIN 53 185.

Der Phosphorgehalt aller Proben wurde durch Röntgenfluoreszenzanalyse (RFA) bestimmt.

Der Wassergehalt der Katalysatorlösungen wurde mittels Karl-Fischer-Titration nach DIN DIN 51777-2 bestimmt.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skale wurden geringe Mengen von Tetramethylsilan im Lösungsmittel mit 0 ppm ¹H-NMR-chem. Verschiebung heran gezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 896 009.

Die dynamischen Viskositäten wurden bei 23 °C mit dem Viskosimeter VT 550 der Fa. Haake bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen. Die Herstellung der Hydrogenpolyfluoridkatalysatoren wird u.a. in EP-A 962454 und darin zit. Lit. beschrieben.

### Beispiel 1 (Vergleichsbeispiel)

In einem doppelwandigen, durch einen externen Kreislauf auf 60°C temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurden 603 mg einer ca. 70%igen isopropanolischen Tetrabutylphosphoniumhydrogendifluoridlösung (Wassergehalt: 0,8%, Phosphorgehalt 7,6%) portionsweise so dosiert, dass die Temperatur der Reaktionsmischung 62°C nicht überschritt. Nachdem ca. 1 mol NCO Gruppen umgesetzt waren, wurde der Katalysator durch Zugabe einer zum Katalysator äquivalenten Menge an p-Toluolsulfonsäure-Monohydrat (als ca. 40%ige Lösung in Isopropanol, Wassergehalt 5,2%) deaktiviert, weitere 30 min bei Reaktionstemperatur nachgerührt und anschließend aufgearbeitet. Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 140°C, KWV-Temp.: 120°C), wobei nicht umgesetztes Monomer als Destillat und das monomerenarme Polyisocyanatharz als Sumpfprodukt separiert wurden (Startdurchlauf, Beispiel 1-A). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde insgesamt fünfmal wiederholt (Katalysatordosen: 530 mg; 498 mg; 492 mg; 486 mg und 466 mg). Der Phosphorgehalt des am Ende der Versuchsserie verbleibenden Recyclatmonomers belief sich auf 78 ppm. Die gemittelten Daten der in den Versuchen 1-B bis 1-F erhaltenen Polyisocyanatharze lauten wie folgt:
Harzausbeute (bezogen auf eingesetztes HDI): 18,2%
NCO-Gehalt: 23,3 %
Viskosität: 720 mPas/23°C
Iminooxadiazindione: 49 mol-%*
Isocyanurate: 46 mol-%*
Uretdione: 5 mol-%*
* = bezogen auf die Summe der in der Modifizierungsreaktion gebildeten NCO-Folgeprodukte

### Beispiel 2 (erfindungsgemäß)

Es wurde Verfahren wie im Vergleichsbeispiel 1 beschrieben, mit dem Unterschied, dass der Wassergehalt des verwendeten Katalysators vorher durch Zugabe einer dem Wassergehalt äquimolaren Menge an Trimethylorthoacetat zur Katalysatorlösung auf 820 ppm abgesenkt worden war und an Stelle der wasserhaltigen Toluolsulfonsäure eine durch Azeotropdestillation mit Toluol auf 0,28% Wassergehalt abgereicherte, Lösung der Sulfonsäure (äquimolar auf Katalysator) in Toluol/Rest-iso-Propanol zum Einsatz kam.

Der Phosphorgehalt des am Ende der Versuchsserie verbleibenden Recyclatmonomers belief sich auf 35 ppm. Die gemittelten Daten der in den Versuchen 2-B bis 2-F erhaltenen Polyisocyanatharze lauten wie folgt:
Harzausbeute (bezogen auf eingesetztes HDI): 18,5 %
NCO-Gehalt: 23,5 %
Viskosität: 685 mPas/23°C
Iminooxadiazindione: 52 mol-%*
Isocyanurate: 43 mol-%*
Uretdione: 5 mol-%*
* = bezogen auf die Summe der in der Modifizierungsreaktion gebildeten NCO-Folgeprodukte

### Beispiel 3 (erfindungsgemäß)

Es wurde Verfahren wie im Beispiel 2 beschrieben, mit dem Unterschied, dass der Wassergehalt des verwendeten Katalysators vorher durch Zugabe einer dem Wassergehalt äquimolaren Menge an Triethylorthoacetat zur Katalysatorlösung auf 1010 ppm abgesenkt worden war.

Der Phosphorgehalt des am Ende der Versuchsserie verbleibenden Recyclatmonomers belief sich auf 32 ppm. Die gemittelten Daten der in den Versuchen 3-B bis 3-F erhaltenen Polyisocyanatharze lauten wie folgt:
Harzausbeute (bezogen auf eingesetztes HDI): 19,0 %
NCO-Gehalt: 23,4 %
Viskosität: 715 mPas/23°C
Iminooxadiazindione: 53 mol-%*
Isocyanurate: 42 mol-%*
Uretdione: 5 mol-%*
* = bezogen auf die Summe der in der Modifizierungsreaktion gebildeten NCO-Folgeprodukte

### Beispiel 4 (erfindungsgemäß)

Es wurde Verfahren wie im Beispiel 3 beschrieben, mit dem Unterschied, dass an Stelle der (nahezu) wasserfreien Toluolsulfonsäure Dodecylbenzolsulfonsäure (Wassergehalt: 0,25%) zum Einsatz kam. Der Phosphorgehalt des am Ende der Versuchsserie verbleibenden Recyclatmonomers belief sich auf 32 ppm. Die gemittelten Daten der in den Versuchen 3-B bis 3-F erhaltenen Polyisocyanatharze lauten wie folgt:
Harzausbeute (bezogen auf eingesetztes HDI): 18,9 %
NCO-Gehalt: 23,5 %
Viskosität: 720 mPas/23°C
Iminooxadiazindione: 53 mol-%*
Isocyanurate: 42 mol-%*
Uretdione: 5 mol-%*
* = bezogen auf die Summe der in der Modifizierungsreaktion gebildeten NCO-Folgeprodukte

## Patentansprüche

1. Katalysator-Kit, umfassend einen Trimerisierungskatalysator für die asymmetrische Trimerisierung von Polyisocyanaten und ein Katalysatorgift des Trimerisierungskatalysators, **dadurch gekennzeichnet, dass** der Trimerisierungskatalysator und das Katalysatorgift jeweils einen Wassergehalt von höchstens 0,5 Gew.-% aufweisen und der Trimerisierungskatalysator wenigstens ein quarternäres Phosphoniumsalz umfasst oder daraus besteht.

2. Katalysator-Kit nach Anspruch 1, **dadurch gekennzeichnet, dass** der Trimerisierungskatalysator und das Katalysatorgift jeweils unabhängig voneinander einen Wassergehalt von höchstens 0,4 Gew.-% aufweisen, bevorzugt von höchstens 0,3 Gew.-% und besonders bevorzugt von höchstens 0,2 Gew.-%.

3. Katalysator-Kit nach Anspruch 1 oder 2, **dadurch gekennzeichnet, dass** das quarternäre Phosphoniumsalz die allgemeine Formel R₄P⁺X⁻ aufweist, wobei die Reste R jeweils unabhängig voneinander gleiche oder verschiedene, gegebenenfalls verzweigte, aliphatische, aromatische und/oder araliphtische C1-C20 Reste sind und / oder wenigstens jeweils zwei der Reste R gemeinsam mit dem Phosphoratom gesättigte oder ungesättigte Cyclen bilden und X ein Halogen ist.

4. Katalysator-Kit nach Anspruch 1 oder 2, **dadurch gekennzeichnet, dass** quarternäre Phosphoniumsalz die allgemeine Formel R₄P⁺ F⁻·n(HF) aufweist, wobei n einen Wert zwischen 0,1 und 20 hat, und wobei die Reste R jeweils unabhängig voneinander gleiche oder verschiedene, gegebenenfalls verzweigte, aliphatische, aromatische und/oder araliphtische C1-C20 Reste sind und / oder wenigstens jeweils zwei der Reste R gemeinsam mit dem Phosphoratom gesättigte oder ungesättigte Cyclen bilden.

5. Katalysator-Kit nach einem der vorstehenden Ansprüche, **dadurch gekennzeichnet, dass** das Katalysatorgift ausgewählt ist aus wasserfreien Säuren mit einem pKs-Wert von 3,2 oder weniger, insbesondere aus wasserfreier HCl, wasserfreien phosphor- und schwefelhaltigen Säuren, deren Estern, deren Säurehalogeniden sowie Kombinationen von diesen.

6. Verwendung eines Katalysator-Kits nach einem der Ansprüche 1 bis 5 zur Herstellung von Iminooxadiazindiongruppen enthaltenden Polyisocyanaten durch Oligomerisierung monomerer Di- und/oder Triisocyanate.

7. Verfahren zur Herstellung von Iminooxadiazindiongruppen enthaltenden Polyisocyanaten, bei dem mindestens ein organisches Di- und/oder Triisocyanat unter Anwesenheit wenigstens eines Trimerisierungskatalysators für die asymmetrische Trimerisierung von Polyisocyanaten zur Reaktion gebracht und die Reaktion bei Erreichen eines vorgebbaren Umsetzungsgrades bezogen auf das organische Di- und/oder Triisocyanat durch Zugabe wenigstens eines Katalysatorgifts für den Trimerisierungskatalysator gestoppt wird,
**dadurch gekennzeichnet, dass** der Trimerisierungskatalysator und das Katalysatorgift jeweils einen Wassergehalt von höchstens 0,5 Gew.-% aufweisen und der Gesamtgehalt an Wasser aller Einsatzstoffe insgesamt höchstens 1 Gew.-% beträgt und der Trimerisierungskatalysator wenigstens ein quarternäres Phosphoniumsalz umfasst oder daraus besteht.

8. Verfahren nach Anspruch 7, **dadurch gekennzeichnet, dass** der Umsetzungsgrad 5 bis 80 Gew.-% des organischen Di- und/oder Triisocyanates ist, insbesondere 10 bis 60 Gew.-% des organischen Di- und/oder Triisocyanates.

9. Verfahren nach Anspruch 7 oder 8, **dadurch gekennzeichnet, dass** die Reaktion bei einer Temperatur von 0 °C bis + 250 °C durchgeführt wird, insbesondere bei einer Temperatur von 20 °C bis + 180°C, vorzugsweise bei einer Temperatur von 40 °C bis + 150°C.

10. Verfahren nach einem der Ansprüche 7 bis 9, **dadurch gekennzeichnet, dass** der Trimerisierungskatalysator in einer Menge von 1 mol-ppm bis 1 mol-% bezogen auf das organische Di- und/oder Triisocyanat eingesetzt wird, insbesondere in einer Menge von 5 mol-ppm bis 0,1 mol-%.

11. Verfahren nach einem der Ansprüche 7 bis 10, **dadurch gekennzeichnet, dass** die Reaktion unter Anwesenheit wenigstens eines Lösemittels und/ oder eines Additivs durchgeführt wird.

12. Verfahren nach einem der Ansprüche 7 bis 11, **dadurch gekennzeichnet, dass** der Trimerisierungskatalysator vor der Zugabe zu dem organischen Di- und/oder Triisocyanat in einem Lösemittel gelöst wird, welches insbesondere ausgewählt ist aus aliphatischen Kohlenwasserstoffen, aromatischen Kohlenwasserstoffen, Alkoholen, Ketonen, Estern, Ethern oder Kombinationen von diesen.

13. Verfahren nach einem der Ansprüche 7 bis 12, **dadurch gekennzeichnet, dass** nach Erreichen des Umsetzungsgrades nicht umgesetztes organisches Di- und/oder Triisocyanat sowie gegebenenfalls verwendetes Lösungsmittel abgetrennt wird, wobei der nach der Abtrennung verbleibende Gehalt an nicht umgesetztem organischen Di- und/oder Triisocyanat insbesondere weniger als 0,5 Gew.-% beträgt, vorzugsweise weniger als 0,3 Gew.-%.

14. Verfahren nach einem der Ansprüche 7 bis 13, **dadurch gekennzeichnet, dass** die Reaktion in einem Rohrreaktor erfolgt.

## Claims

1. Catalyst kit comprising a trimerization catalyst for asymmetric trimerization of polyisocyanates and a catalyst poison for the trimerization catalyst, **characterized in that** the trimerization catalyst and the catalyst poison each have a water content of not more than 0.5% by weight and the trimerization catalyst comprises or consists of at least one quaternary phosphonium salt.

2. Catalyst kit according to Claim 1, **characterized in that** the trimerization catalyst and the catalyst poison each independently of one another have a water content of not more than 0.4% by weight, preferably of not more than 0.3% by weight and particularly preferably of not more than 0.2% by weight.

3. Catalyst kit according to Claim 1 or 2, **characterized in that** the quaternary phosphonium salt has the general formula R₄P⁺X⁻, wherein the radicals R are each independently of one another identical or different, optionally branched, aliphatic, aromatic and/or araliphatic C1-C20 radicals and/or in each case at least two of the radicals R form saturated or unsaturated cycles in conjunction with the phosphorus atom and X is a halogen.

4. Catalyst kit according to Claim 1 or 2, **characterized in that** quaternary phosphonium salt has the general formula R₄P⁺F⁻·n(HF), wherein n has a value between 0.1 and 20, and wherein the radicals R are each independently of one another identical or different, optionally branched, aliphatic, aromatic and/or araliphatic C1-C20 radicals and/or in each case at least two of the radicals R form saturated or unsaturated cycles in conjunction with the phosphorus atom.

5. Catalyst kit according to any of the preceding claims, **characterized in that** the catalyst poison is selected from anhydrous acids having a pKₐ of not more than 3.2, in particular from anhydrous HCl, anhydrous phosphorus- and sulfur-containing acids, esters thereof, acid halides thereof and combinations thereof.

6. Use of a catalyst kit according to any of Claims 1 to 5 for producing iminooxadiazinedione-containing polyisocyanates by oligomerization of monomeric di-and/or triisocyanates.

7. Process for producing iminooxadiazinedione-containing polyisocyanates, wherein at least one organic di- and/or triisocyanate is reacted in the presence of at least one trimerization catalyst for asymmetric trimerization of polyisocyanates and upon achieving a predeterminable degree of conversion based on the organic di- and/or triisocyanate the reaction is terminated by addition of at least one catalyst poison for the trimerization catalyst, **characterized in that** the trimerization catalyst and the catalyst poison each have a water content of not more than 0.5% by weight and the total water content of all starting materials altogether is not more than 1% by weight and the trimerization catalyst comprises or consists of at least one quaternary phosphonium salt.

8. Process according to Claim 7, **characterized in that** the degree of conversion is 5% to 80% by weight of the organic di- and/or triisocyanate, in particular 10% to 60% by weight of the organic di-and/or triisocyanate.

9. Process according to Claim 7 or 8, **characterized in that** the reaction is performed at a temperature of 0°C to +250°C, in particular at a temperature of 20°C to +180°C, preferably at a temperature of 40°C to +150°C.

10. Process according to any of Claims 7 to 9, **characterized in that** the trimerization catalyst is employed in an amount of 1 mol ppm to 1 mol% based on the organic di- and/or triisocyanate, in particular in an amount of 5 mol ppm to 0.1 mol%.

11. Process according to any of Claims 7 to 10, **characterized in that** the reaction is performed in the presence of at least one solvent and/or one additive.

12. Process according to any of Claims 7 to 11, **characterized in that** prior to addition to the organic di- and/or triisocyanate the trimerization catalyst is dissolved in a solvent which is especially selected from aliphatic hydrocarbons, aromatic hydrocarbons, alcohols, ketones, esters, ethers or combinations thereof.

13. Process according to any of Claims 7 to 12, **characterized in that** after achieving the degree of conversion unconverted organic di- and/or triisocyanate and any solvent employed is removed, wherein the content of unconverted organic di-and/or triisocyanate remaining after the removal is in particular less than 0.5% by weight, preferably less than 0.3% by weight.

14. Process according to any of Claims 7 to 13, **characterized in that** the reaction is carried out in a tubular reactor.

## Revendications

1. Kit de catalyseur, comprenant un catalyseur de trimérisation pour la trimérisation asymétrique de polyisocyanates et un poison de catalyseur du catalyseur de trimérisation, **caractérisé en ce que** le catalyseur de trimérisation et le poison de catalyseur présentent à chaque fois une teneur en eau d'au plus 0,5 % en poids et le catalyseur de trimérisation comprend au moins un sel de phosphonium quaternaire ou en est constitué.

2. Kit de catalyseur selon la revendication 1, **caractérisé en ce que** le catalyseur de trimérisation et le poison de catalyseur présentent à chaque fois indépendamment l'un de l'autre une teneur en eau d'au plus 0,4 % en poids, préférablement d'au plus 0,3 % en poids et particulièrement préférablement d'au plus 0,2 % en poids.

3. Kit de catalyseur selon la revendication 1 ou 2, **caractérisé en ce que** le sel de phosphonium quaternaire présente la formule générale R₄P⁺X⁻, les radicaux R, à chaque fois indépendamment les uns des autres identiques ou différents, étant des radicaux aliphatiques, aromatiques et/ou araliphatiques, éventuellement ramifiés, en C₁₋₂₀ et/ou au moins à chaque fois deux des radicaux R formant conjointement avec l'atome de phosphore des cycles saturés ou insaturés et X étant un halogène.

4. Kit de catalyseur selon la revendication 1 ou 2, **caractérisé en ce que** le sel de phosphonium quaternaire présente la formule générale R₄P⁺F⁻·n(HF), n possédant une valeur entre 0,1 et 20, et les radicaux R, à chaque fois indépendamment les uns des autres identiques ou différents, étant des radicaux aliphatiques, aromatiques et/ou araliphatiques, éventuellement ramifiés, en C₁₋₂₀ et/ou au moins à chaque fois deux des radicaux R formant conjointement avec l'atome de phosphore des cycles saturés ou insaturés.

5. Kit de catalyseur selon l'une quelconque des revendications précédentes, **caractérisé en ce que** le poison de catalyseur est choisi parmi des acides anhydres dotés d'une valeur de pKs de 3,2 ou moins, en particulier parmi HCl anhydres, des acides anhydres contenant du phosphore et des acides anhydres contenant du soufre, leurs esters, leurs halogénures d'acide ainsi que des combinaisons de ceux-ci.

6. Utilisation d'un kit de catalyseur selon l'une quelconque des revendications 1 à 5 pour la préparation de polyisocyanates contenant des groupes iminooxadiazinedione par oligomérisation de diisocyanates et/ou de triisocyanates monomériques.

7. Procédé pour la préparation de polyisocyanates contenant des groupes iminooxadiazinedione dans lequel au moins un diisocyanate et/ou un triisocyanate organiques sont mis en réaction en présence d'au moins un catalyseur de trimérisation pour la trimérisation asymétrique de polyisocyanates et la réaction est stoppée, à l'atteinte d'un degré de transformation prédéfinissable par rapport au diisocyanate et/ou au triisocyanate organiques, par l'ajout d'au moins un poison de catalyseur pour le catalyseur de trimérisation,
**caractérisé en ce que** le catalyseur de trimérisation et le poison de catalyseur présentent à chaque fois une teneur en eau d'au plus 0,5 % en poids et la teneur totale en eau de toutes les substances utilisées est au total d'au plus 1 % en poids et le catalyseur de trimérisation comprend au moins un sel de phosphonium quaternaire ou en est constitué.

8. Procédé selon la revendication 7, **caractérisé en ce que** le degré de transformation est de 5 à 80 % en poids du diisocyanate et/ou du triisocyanate organiques, en particulier 10 à 60 % en poids du diisocyanate et/ou du triisocyanate organiques.

9. Procédé selon la revendication 7 ou 8, **caractérisé en ce que** la réaction est mise en œuvre à une température de 0 °C à + 250 °C, en particulier à une température de 20 °C à + 180 °C, de préférence à une température de 40 °C à + 150 °C.

10. Procédé selon l'une quelconque des revendications 7 à 9, **caractérisé en ce que** le catalyseur de trimérisation est utilisé en une quantité de 1 ppm en moles à 1 % en moles par rapport au diisocyanate et/ou au triisocyanate organiques, en particulier en une quantité de 5 ppm en moles à 0,1 % en moles.

11. Procédé selon l'une quelconque des revendications 7 à 10, **caractérisé en ce que** la réaction est mise en œuvre en présence d'au moins un solvant et/ou d'au moins un additif.

12. Procédé selon l'une quelconque des revendications 7 à 11, **caractérisé en ce que** le catalyseur de trimérisation est dissous dans un solvant avant l'ajout au diisocyanate et/ou au triisocyanate organiques, qui est en particulier choisi parmi des hydrocarbures aliphatiques, des hydrocarbures aromatiques, des alcools, des cétones, des esters, des éthers et des combinaisons de ceux-ci.

13. Procédé selon l'une quelconque des revendications 7 à 12, **caractérisé en ce qu'**après l'atteinte du degré de transformation, le diisocyanate et/ou le triisocyanate organiques n'ayant pas réagi ainsi qu'éventuellement le solvant utilisé sont séparés, la teneur restante, après la séparation, de diisocyanate et/ou de triisocyanate organiques n'ayant pas réagi étant en particulier inférieure à 0,5 % en poids, de préférence inférieure à 0,3 % en poids.

14. Procédé selon l'une quelconque des revendications 7 à 13, **caractérisé en ce que** la réaction est réalisée dans un réacteur tubulaire.
